# EUROPÄISCHE PATENTSCHRIFT

(11) **EP 1 110 578 B1**
(45) Veröffentlichungstag und Bekanntmachung des Hinweises auf die Patenterteilung: **23.11.2005**
(21) Anmeldenummer: 00250430.6
(22) Anmeldetag: 12.12.2000
(51) Int. Cl.: A61N 1/05

(54) **Zwei-Kammer Single-Pass Elektroden-Anordnung**
Dual chamber single-pass electrode assembly
Ensemble d'électrodes double chambre à passage unique

(30) Priorität: 23.12.1999 DE 19963602
(43) Veröffentlichungstag der Anmeldung: 27.06.2001
(73) Patentinhaber: BIOTRONIK Mess- und Therapiegeräte GmbH & Co Ingenieurbüro Berlin, 12359 Berlin (DE)
(72) Erfinder: Audoglio, Roberto, 27010 Linarolo (IT)
(74) Vertreter: Eisenführ, Speiser & Partner

(56) Entgegenhaltungen:
- WO-A-99/03530
- US-A- 4 332 259
- US-A- 5 111 811
- US-A- 5 628 779
- US-A- 5 755 766

## Beschreibung

Die Erfindung betrifft eine Elektrodenanordnung mit einem zum Einführen in den Coronarsinus ausgebildeten ersten Elektrodenkabel, das einen Elektrodenleiter und mindestens eine Elektrode zur Abgabe und/oder Aufnahme elektrischer Signale im Coronarsinus aufweist.

Die Herzstimulation über den Coronarsinus (CS) ist heute eine selten angewendete Therapieform. Ursache hierfür ist neben der schwierigen Positionierung der Elektroden und neben drohenden Komplikationen wie Perforation auch das Fehlen geeigneter Elektrodenanordnungen.

Es sind jedoch Fälle bekannt, für die die CS-Stimulation eine bevorzugte Therapieform darstellt, etwa bei der Implantation antitachykarder Schrittmacher, bei der die Stimulationselektrode nah am Reentry-Kreis liegen soll. Auch eine Stimulation der linken Herzhälfte über den CS und eine Herzvene ist denkbar.

Aufgabe der Erfindung ist es daher, eine Elektrodenanordnung für eine elektrische Therapie des Herzens anzugeben.

Die Aufgabe wird durch eine Elektrodenanordnung mit den Merkmalen des Anspruchs 1 gelöst.

Erfindungsgemäß ist im Lumen des ersten Elektrodenkabels zumindest längsabschnittsweise ein zweites Elektrodenkabel längsverschieblich führbar, das durch eine Öffnung am distalen Ende des ersten Elektrodenkabels aus diesem heraustreten kann und zum Einführen in eine in den Coronarsinus mündende Herzvene ausgebildet ist.

Die erfindungsgemäße Elektrodenanordnung ist in erster Linie für eine Stimulation der linken Herzhälfte über den Coronarsinus (CS) und eine linke Herzvene ausgelegt. Ihre Konstruktion ermöglicht ein relativ einfaches Einführen der Elektroden in die Gefäße. Für die Einführung beider Elektrodenkabel ist nur eine Gefäßinzision erforderlich, beispielsweise in der Vena cephalica oder der Vena subclavia oder anderen Gefäßen. Dabei kann zunächst das erste Elektrodenkabel eingeführt und im Koronarsinus plaziert werden. Anschließend kann das zweite Elektrodenkabel durch das Lumen des ersten Elektrodenkabels geführt und aus dessen distalem Ende heraustretend in eine Herzvene eingeführt werden.

Nach der Positionierung des ersten Elektrodenkabels kann sein Lumen alternativ zunächst für das Einführen eines Swan-Ganz-Katheters genutzt werden, um ein Röntgenkontrastmittel in die Herzvenen zu injizieren. Dadurch können dann mit Hilfe der Fluoroskopie die Herzvenen sichtbar gemacht werden. Dieses Vorgehen erleichtert das anschließend erforderliche, präzise Positionieren des zweiten Elektrodenkabels in der Herzvene.

Das zweite Elektrodenkabel kann aber alternativ auch bereits beim Einführen des ersten Elektrodenkabels bis zu dessen distaler Öffnung eingeschoben sein.

Die Elektrode des ersten Elektrodenkabels ist vorzugsweise als Ringelektrode ausgebildet. Bei einer bevorzugten Ausführungsform der Erfindung weist das erste Elektrodenkabel zwei axial beabstandete, im CS positionierbare Ringelektroden auf. Diese sind vorzugsweise gegeneinander isoliert und entgegengesetzt polarisierbar.

Eine Fixierung des ersten Elektrodenkabels im Coronarsinus kann sowohl aktiv als auch passiv erfolgen, wobei auf eine nur minimale Stauwirkung der Verankerungsvorrichtung zu achten ist. Beispielsweise kann eine Verankerungsvorrichtung als Schneckengewindeabschnitt aus einem flexiblem Kunststoffmaterial ausgebildet und auf dem Außenmantel des ersten Elektrodenkabels im Bereich seines distalen Endes angeordnet sein. Denkbar ist auch eine Fixierung mit Hilfe dem CS entsprechend dimensionierter, zum proximalen Ende hin geneigter Finnen aus elastischem Kunststoffmaterial. Zur Verringerung des Strömungswiderstandes einer solchen Fixierungsvorrichtung können sich die sich Finnen jeweils nur über einen Abschnitt des Mantelumfangs erstrecken und sowohl in axialer wie in Umfangsrichtung versetzt angeordnet sein.

Das erste Elektrodenkabel hat in einer Weiterbildung der Erfindung einen Außenmantel mit einer Aufnahmeöffnung für das zweite Elektrodenkabel. Zwischen der Aufnahmeöffnung und der distalen Elektrode ist das Lumen des ersten Elektrodenkabels so weit vergrößert, daß das zweite Elektrodenkabel entlang des Elektrodenleiters des ersten Elektrodenkabels geführt werden kann.

Bei einer weiteren Ausführungsform ist eine Arretierung der axialen Position des zweiten Elektrodenkabels relativ zum Außenmantel möglich. Auf diese Weise wird nach der Positionierung der Elektroden im CS und in der Herzvene eine Veränderung des Abstandes der Herzvenenelektrode zur bzw. zu den CS-Elektroden verhindert.

Die Arretierung wird vorzugsweise mit Hilfe einer Klemmvorrichtung an der Aufnahmeöffnung erzielt, etwa mit einer sich im Lumen des Außenmantels senkrecht zur Längsachse zwischen der ersten und der zweiten Elektrodenleitung erstreckenden Exzenterschraube.

Hierfür ist das Querschnittsprofil des Außenmantels an der Aufnahmeöffnung verbreitert. Dadurch wird gleichzeitig das Einführen des zweiten Elektrodenkabels erleichtert.

Eine Dichtung an der distalen Öffnung des ersten Elektrodenkabels verhindert den Eintritt von Flüssigkeit in das Lumen des Außenmantels. Das Lumen ist vorzugsweise auch abgedichtet, solange das zweite Elektrodenkabel noch nicht in die Herzvene eingeführt ist.

Das zweite Elektrodenkabel verjüngt sich bei einem weiteren Ausführungsbeispiel zu seinem distalen Ende hin, um einen geringen Strömungswiderstand zu bilden.

Zur Stimulation des linken Ventrikels wird vorzugsweise eine Spitzenelektrode am distalen Ende des zweiten Elektrodenkabels verwendet. Aber auch Elektrodenanordnungen mit einer oder mehreren Ringelektroden in dem aus dem Außenmantel des ersten Elektrodenkabels nach distal herausragenden Abschnitt des zweiten Elektrodenkabels sind grundsätzlich möglich.

Bei einem weiteren Ausführungsbeispiel ist eine Vorrichtung zur Fixierung des zweiten Elektrodenkabels in der Herzvene vorgesehen. Sie kann, bei entsprechender Dimensionierung, ähnlich ausgebildet werden wie die Fixierungsvorrichtung für den CS.

Am distalen Ende des zweiten Elektrodenkabels (14) kann ein Fortsatz mit mindestens einem Marker angeordnet sein. Der Marker kann mittels eines bildgebenden Verfahrens von außerhalb des Körpers geortet werden. Beispielsweise enthält der Makrer ein Material oder ist vollständig aus einem Material gefertigt, mit dem in einem durch das bildgebende Verfahren erzeugten Bild ein Kontrast zu körpereigenen Materialien erzeugbar ist. Für eine Röntgendurchstrahlung des Patienten zur Überwachung der Einführung der Elektroden kann der Marker beispielsweise Gold enthalten. Ein oder mehrere solche Marker können auch im distalen Endbereich des ersten Elektrodenkabels vorgesehen sein.

Der Fortsatz ist aus flexiblem Material gefertigt kann insbesondere steuerbar ausgeführt werden, beispielsweise, indem der Führungsdraht für das zweite Elektrodenkabel in ihn einführbar ist. Es ist aber auch eine Ausbildung denkbar, in der der Fortsatz etwa J- oder hornförmig ist und beim Vorschub in den Gefäßen durch Drehung des zweiten Elektrodenkabels gelenkt wird.

Zur Erzielung einer hohen Bruchfestigkeit ist der Elektrodenleiter des ersten Elektrodenkabels zweiwendlig ausgeführt. In jeder der beiden Wendeln des Elektrodenleitersverlaufen bei einem Ausführungsbeispiel mit zwei CS-Elektroden zweijeweils elektrisch isolierte Drähte.

Zur Einführung und Positionierng der Elektrodenkabel ist bei einer weiteren Ausführungsform je ein Führungsdraht in das Lumen des ersten und des zweiten Elektrodenleiters einführbar.

Um ausreichend Spiel bei der Positionierung des zweiten Elektrodenkabels und bei der Festlegung des Abstandes zwischen der Herzvenenelektrode und den CS-Elektroden zur Verfügung zu stellen, kann das das zweite Elektrodenkabel zwischen seinem proximalen Ende und dem Eintritt in die Aufnahmeöffnung des ersten Elektrodenkabels als ringförmige Schlaufe verlaufen.

Weitere Vorteile der Erfindung werden bei der nachfolgenden Beschreibung eines bevorzugten Ausführungsbeispiels der Erfindung anhand der Zeichnung deutlich.

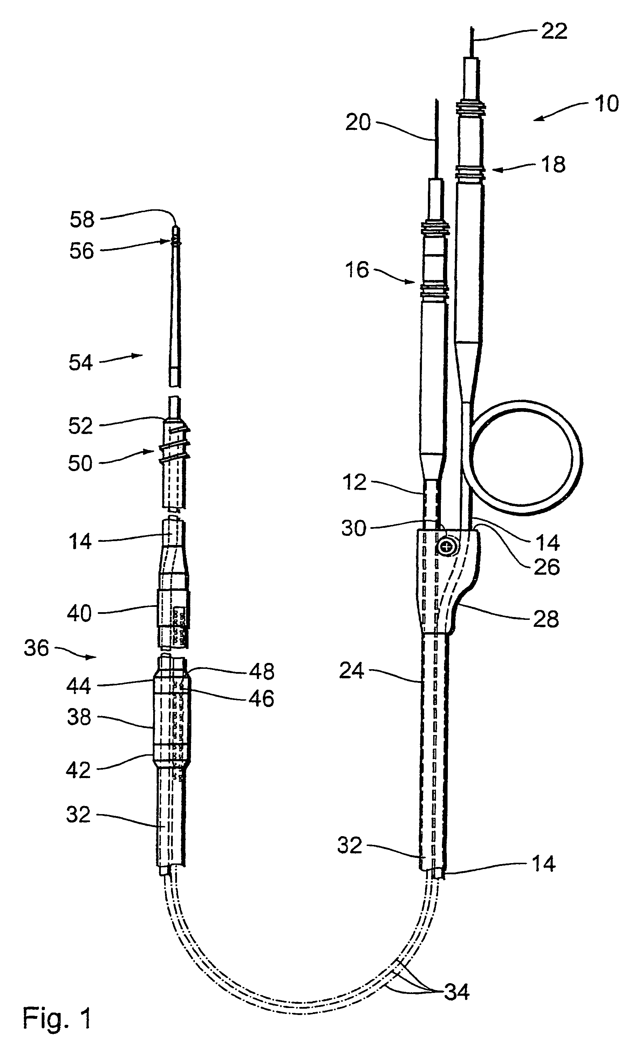

Die einzige Figur 1 zeigt eine vereinfachte, teilweise geschnittene Ansicht einer Elektrodenanordnung für die CS- und LV-Stimulation.

Die Elektrodenanordnung 10 dient zur Stimulation des linken Vorhofs über den Coronarsinus und zur Stimulation des linken Ventrikels über eine in den Coronarsinus mündende Herzvene und weist hierfür zwei Elektrodenkabel (Schrittmachersonden) 12 und 14 auf. Das erste Elektrodenkabel 12 ist an seinem proximalen Ende mit Hilfe eines bipolaren IS-1-Steckers mit einem nicht dargestellten Herzschrittmacher verbindbar. Das zweite Elektrodenkabel 14 hat hierfür an seinem proximalen Ende einen unipolaren IS-1-Stecker.

Die proximalen Endflächen der Stecker 16 und 18 weisen je eine zentrische Öffnung zum Einführen eines Führungsdrahtes (Mandrins) 20 bzw. 22 auf. Die Führungsdrähte 20 und 22 können von den Steckern 16 und 18 her im Lumen der Elektrodenkabel 12 und 14 bis zum jeweiligen distalen Ende geführt werden. Mit Hilfe der der steuerbaren Führungsdrähte 20 und 22 können beide Elektrodenkabel von einer Inzisionsstelle in ihre endgültige Position vorgeschoben werden.

Das erste Elektrodenkabel 12 hat in einem Längsabschnitt, der sich von jenseits des Steckers 16 bishin zum distalen Ende des ersten Elektrodenkabels 12 erstreckt, einen Außenmantel 24. Dessen Lumen nimmt zum einen das erste Elektrodenkabel 12 selbst auf und erlaubt zum anderen das Durchführen des zweiten Elektrodenkabels 14, das an einer Öffnung 26 in ein Aufnahmestück 28 des Außenmantels 24 einführbar ist. Die Elektrodenanordnung des vorliegenden Ausführungsbeispiels ist also eine sogenannte "Single Pass Dual Lead"-Anordnung.

Das Aufnahmestück 28 ist von distal her zur Öffnung 26 hin verbreitert und weist eine etwa mittig angeordnete Arretierschraube 30 auf, die sich quer durch das Lumen hindurch zwischen dem ersten und dem zweiten Elektrodenkabel erstreckt. Die Arretierschraube 30 ist eine Exzenterschraube und kann in eine Stellung gedreht werden, in der sie das zweite Elektrodenkabel 14 gegen die Innenwand des Aufnahmestücks drückt und dadurch zwischen sich und der Innenwand festklemmt.

An das Aufnahmestück 28 schließt sich nach distal hin ein Abschnitt des ersten Elektrodenkabels 12 mit gleichbleibendem Querschnitt an. Das erste und zweite Elektrodenkabel 12 und 14 verlaufen nebeneinander im Lumen des Außenmantels 24. Der Außenmantel und die Ummantelungen des darin geführten ersten Elektrodenleiters 32 sowie des zweiten Elektrodenkabels 14 sind aus reibungsarmen Materialien gefertigt, so daß beim Einführen des zweiten Elektrodenkabels 14 nur geringe axiale Kräfte auftreten und eine Dislokation der unten näher beschriebenen Elektroden vermieden wird.

In der Figur 1 ist die Elektrodenanordnung 10 nicht in ihrer gesamten Länge dargestellt. Gestrichelte, bogenförmige Linien 34 symbolisieren die Fortsetzung des Abschnitts des ersten Elektrodenkabels 12 mit gleichbleibendem Querschnitt bis hin zum im folgenden beschriebenen, distalen Abschnitt 36 der Elektrodenanordnung 10.

Im distalen Abschnitt 36 sind zwei axial beabstandete, ringförmige CS-Elektroden 38 und 40 vorgesehen, die den Außenmantel 24 umspannen. Die axiale Erstrekkung der proximalen Ringelektrode 38 ist etwas größer als die der distalen Ringelektrode 40. Im Bereich der axialen Erstreckung der Ringelektroden 38 und 40 ist das Lumen des Außenmantels 24 gegen Flüssigkeitseintritt abgedichtet und elektrisch isoliert. Hierzu ist die proximale Ringelektrode 40 in axialer Richtung zwischen zwei Isolationsringen 42 und 44 positioniert.

Die elektrische Versorgung der Ringelektroden 38 und 40 erfolgt mit Hilfe des Elektrodenleiters 32, der zweiwendlig ausgeführt ist. Beide Leiterwendeln 46 und 48 (hier geschnitten dargestellt) enthalten zwei jeweils elektrisch isolierte Drähte (nicht dargestellt) und sind nebeneinander gewickelt. Zur elektrischen Isolation sind die Drähte mit PFE beschichtet.

Der erste Elektrodenleiter 34 endet im Bereich der distalen Ringelektrode 40. Von hier an verjüngt sich der Außenmantel 24 des ersten Elektrodenkabels 12 nach distal hin. In seinem Lumen wird lediglich noch das zweite Elektrodenkabel 14 geführt.

Am distalen Ende des ersten Elektrodenkabels 12 ist eine CS-Fixierungsvorrichtung 50 in Form eines Schneckengewindeabschnitts mit zweieinhalb Windungen angeordnet.

Die distale Spitze des ersten Elektrodenkabels 12 ist mit Hilfe einer Dichtung 52, die zwei O-Ringe aufweist, gegen den Eintritt von Flüssigkeit geschützt. Diese Dichtung erlaubt das Herausführen des distalen Endes 54 des zweiten Elektrodenkabels aus dem Lumen des Außenmantels 24 des ersten Elektrodenkabels 12 heraus. Wie oben näher beschrieben, kann vor der Einführung der Spitzenelektrode 58 zunächst ein Swan-Ganz-Katheter durch das Lumen des Außenmantels 24 geführt werden, um ein Röntgenkontrastmittel in die Herzvenen zu injizieren. Anschließend wird das Swan-Ganz-Katheter zurückgezogen, wobei sich die Dichtung 52 am distalen Ende 52 des ersten Elektrodenkabels wieder verschließt.

Das zweite Elektrodenkabel 14 verjüngt sich im Abschnitt 54 bishin zu seinem distalen Ende, an dem eine Fixierungsvorrichtung 56 und eine halbkugelförmige Spitzenelektrode 58 angeordnet sind. Die Fixierungsvorrichtung 56 ist in diesem Ausführungsbeispiel ebenfalls als Schneckengewindeabschnitt ausgebildet.

Auch andere Ausführungsformen der Erfindung als die hier beschriebene sind denkbar. So kann auch das zweite Elektrodenkabel bipolar ausgebildet sein. Grundsätzlich ist eine unipolare oder bipolare Ausführung des ersten und/oder des zweiten Elektrodenkabels möglich.

Das zweite Elektrodenkabel kann wie das erste im oben ausführlich beschriebenen Ausführungsbeispiel zweiwendlig ausgeführt werden.

## Patentansprüche

1. Elektrodenanordnung (10) mit einem zum Einführen in den Coronarsinus ausgebildeten ersten Elektrodenkabel (12), das einen Elektrodenleiter (32) und mindestens eine Elektrode (38, 40) zur Abgabe und/oder Aufnahme elektrischer Signale im Coronarsinus aufweist, **dadurch gekennzeichnet, daß** im Lumen des ersten Elektrodenkabels (12) zumindest längsabschnittsweise ein zweites Elektrodenkabel (14) längsverschieblich führbar ist, das durch eine Öffnung (52) am distalen Ende des ersten Elektrodenkabels aus diesem heraustreten kann und zum Einführen in eine in den Coronarsinus mündende Herzvene ausgebildet ist.

2. Elektrodenanordnung nach Anspruch 1, **dadurch gekennzeichnet, daß** die Elektrode (38, 40) des ersten Elektrodenkabels (12) als Ringelektrode ausgebildet ist.

3. Elektrodenanordnung nach Anspruch 1 oder 2, **dadurch gekennzeichnet, daß** das erste Elektrodenkabel (12) zwei axial beabstandete Elektroden (38, 40) aufweist.

4. Elektrodenanordnung nach Anspruch 3, **dadurch gekennzeichnet, daß** die beiden Elektroden (38, 40) voneinander elektrisch isoliert und entgegengesetzt polarisierbar sind.

5. Elektrodenanordnung nach einem der vorstehenden Ansprüche, **dadurch gekennzeichnet, daß** das erste Elektrodenkabel (12) an seinem distalen Ende (52) eine Vorrichtung (50) zur Fixierung im Coronarsinus aufweist.

6. Elektrodenanordnung nach einem der vorstehenden Ansprüche, **dadurch gekennzeichnet, daß** das erste Elektrodenkabel (12) sich hinter der - bzw. hinter der distalen - Elektrode nach distal hin verjüngt.

7. Elektrodenanordnung nach einem der vorstehenden Ansprüche, **dadurch gekennzeichnet, daß** das erste Elektrodenkabel einen Außenmantel (24) mit einer Aufnahmeöffnung (26) für das zweite Elektrodenkabel (14) hat.

8. Elektrodenanordnung nach einem der vorstehenden Ansprüche, **dadurch gekennzeichnet, daß** Mittel (30) zur Arretierung der axialen Position des zweiten Elektrodenkabels (14) relativ zum Außenmantel (24) vorgesehen sind.

9. Elektrodenanordnung nach Anspruch 8, **dadurch gekennzeichnet, daß** die Mittel zur Arretierung eine Klemmvorrichtung (30) enthalten.

10. Elektrodenanordnung nach Anspruch 9, **dadurch gekennzeichnet, daß** die Klemmvorrichtung an der Aufnahmeöffnung (26) vorgesehen ist und eine sich im Lumen des Außenmantels (24) senkrecht zur Längsachse zwischen der ersten (12) und der zweiten Elektrodenleitung (14) erstreckende Exzenterschraube (30) aufweist.

11. Elektrodenanordnung nach Anspruch 10, **dadurch gekennzeichnet, daß** das Querschnittsprofil des Außenmantels (24) zur Aufnahmeöffnung (26) hin verbreitert ist.

12. Elektrodenanordnung nach einem der vorstehenden Ansprüche, **dadurch gekennzeichnet, daß** das Lumen des ersten Elektrodenkabels (12) im Bereich der Elektrode bzw. der Elektroden (38, 40) gegen Flüssigkeitsein- und austritt sowie elektrisch isoliert ist.

13. Elektrodenanordnung nach Anspruch 12, **gekennzeichnet durch** eine Dichtung (52) gegen den Eintritt von Flüssigkeit an der distalen Öffnung des ersten Elektrodenkabels (12).

14. Elektrodenanordnung nach einem der vorstehenden Ansprüche, **dadurch gekennzeichnet, daß** das zweite Elektrodenkabel (14) sich zu seinem distalen Ende hin verjüngt.

15. Elektrodenanordnung nach einem der vorstehenden Ansprüche, **gekennzeichnet durch** eine Spitzenelektrode (58) am distalen Ende des zweiten Elektrodenkabels (14).

16. Elektrodenanordnung nach einem der vorstehenden Ansprüche, **gekennzeichnet durch** mindestens eine Ringelektrode in dem aus dem Außenmantel des ersten Elektrodenkabels nach distal herausragenden Abschnitt (54) des zweiten Elektrodenkabels (14).

17. Elektrodenanordnung nach einem der vorstehenden Ansprüche, **gekennzeichnet durch** eine Vorrichtung (56) zur Fixierung des zweiten Elektrodenkabels (14) in der Herzvene.

18. Elektrodenanordnung nach einem der vorstehenden Ansprüche, **dadurch gekennzeichnet, daß** am distalen Ende des zweiten Elektrodenkabels (14) ein, insbesondere steuerbarer, Fortsatz mit mindestens einem Marker angeordnet ist, der mittels eines bildgebenden Verfahrens von außerhalb des Körpers ortbar ist.

19. Elektrodenanordnung nach einem der vorstehenden Ansprüche, **gekennzeichnet durch** eine bipolare Ausführung des zweiten Elektrodenkabels (14).

20. Elektrodenanordnung nach einem der vorstehenden Ansprüche, **dadurch gekennzeichnet, daß** der Elektrodenleiter (32) des ersten (12) und/oder des zweiten Elektrodenkabels (14) zweiwendlig ausgeführt ist.

21. Elektrodenanordnung nach Anspruch 20, **dadurch gekennzeichnet, daß** in jeder der beiden Wendeln (46, 48) des Elektrodenleiters (32) zwei jeweils elektrisch isolierte Drähte verlaufen.

22. Elektrodenanordnung nach einem der vorstehenden Ansprüche, **dadurch gekennzeichnet, daß** je ein Führungsdraht (20, 22) in das Lumen des ersten (32) und in das Lumen eines Elektrodenleiters des zweiten Elektrodenkabels (14) einführbar ist.

23. Elektrodenanordnung nach einem der vorstehenden Ansprüche, **dadurch gekennzeichnet, daß** das zweite Elektrodenkabel (14) zwischen seinem proximalen Ende und dem Eintritt in die Aufnahmeöffnung des ersten Elektrodenkabels als ringförmige Schlaufe verläuft.

## Claims

1. Electrode arrangement (10) comprising a first electrode cable (12) designed to be inserted into the coronary sinus, and which comprises an electrode conductor (32) and at least one electrode (38, 40) for outputting and/or receiving electrical signals in the coronary sinus, **characterised in that** in the lumen of the first electrode cable (12) at least in a longitudinal portion-wise manner a second electrode cable (14) can be guided in a longitudinally displaceable manner, which second electrode cable can project through an opening (52) at the distal end of the first electrode cable and which is designed to be inserted into a cardiac vein which opens into the coronary sinus.

2. Electrode arrangement according to claim 1 **characterised in that** the electrode (38, 40) of the first electrode cable (12) is in the form of a ring electrode.

3. Electrode arrangement according to claim 1 or claim 2 **characterised in that** the first electrode cable (12) has two axially spaced electrodes (38, 40).

4. Electrode arrangement according to claim 3 **characterised in that** the two electrodes (38, 40) are electrically insulated from each other and can be oppositely polarised.

5. Electrode arrangement according to one of the preceding claims **characterised in that** the first electrode cable (12) at its distal end (52) has a device (50) for securing in the coronary sinus.

6. Electrode arrangement according to one of the preceding claims **characterised in that** the first electrode cable (12) tapers in a distal direction downstream of the electrode or downstream of the distal electrode.

7. Electrode arrangement according to one of the preceding claims **characterised in that** the first electrode cable has an external casing (24) with a receiving opening (26) for the second electrode cable (14).

8. Electrode arrangement according to one of the preceding claims **characterised in that** means (30) are provided for arresting the axial position of the second electrode cable (14) relative to the external casing (24).

9. Electrode arrangement according to claim 8, **characterised in that** the arresting means include a clamping device (30).

10. Electrode arrangement according to claim 9, **characterised in that** the clamping device is provided at the receiving opening (26) and has an eccentric screw (30) which extends in the lumen of the external casing (24) perpendicularly to the longitudinal axis between the first (12) and the second electrode line (14).

11. Electrode arrangement according to claim 10, **characterised in that** the cross-sectional profile of the external casing (24) has a greater width towards the receiving opening (26).

12. Electrode arrangement according to one of the preceding claims **characterised in that** the lumen of the first electrode cable (12) is electrically insulated and protected from the admission and exodus of fluid in the region of the electrode or electrodes (38, 40).

13. Electrode arrangement according to claim 12, **characterised by** a seal (52) to prevent the admission of fluid at the distal opening of the first electrode cable (12).

14. Electrode arrangement according to one of the preceding claims, **characterised in that** the second electrode cable (14) tapers towards its distal end.

15. Electrode arrangement according to one of the preceding claims, **characterised by** a point electrode (58) at the distal end of the second electrode cable (14).

16. Electrode arrangement according to one of the preceding claims, **characterised by** at least one ring electrode in the section (54) of the second electrode cable (14) projecting distally out of the external casing of the first electrode cable.

17. Electrode arrangement according to one of the preceding claims, **characterised by** a device (56) for fixing the second electrode cable (14) in the cardiac vein.

18. Electrode arrangement according to one of the preceding claims, **characterised in that** at the distal end of the second electrode cable (14) an, in particular controllable, extension with at least one marker is arranged, which can be located by means of an imaging process from outside the body.

19. Electrode arrangement according to one of the preceding claims, **characterised by** a bipolar design of the second electrode cable (14).

20. Electrode arrangement according to one of the preceding claims, **characterised in that** the electrode conductor (32) of the first (12) and/or the second (14) electrode cable is of a two-coil nature.

21. Electrode arrangement according to claim 20, **characterised in that** two respectively electrically insulated wires run in each of the two coils (46, 48) of the electrode conductor (32).

22. Electrode arrangement according to one of the preceding claims, **characterised in that** a respective guide wire (20, 22) can be inserted into the lumen of the first electrode conductor (32) and into the lumen of an electrode conductor of the second electrode cable (14).

23. Electrode arrangement according to one of the preceding claims, **characterised in that** the second electrode cable (14) runs in the form of an annular loop between its proximal end and the entry into the receiving opening of the first electrode cable.

## Revendications

1. Dispositif à électrodes (10) comportant un premier câble d'électrode (12) agencé pour être introduit dans le sinus coronarien et qui comporte un conducteur d'électrode (32) et au moins une électrode (38,40) pour délivrer et/ou enregistrer des signaux électriques dans le sinus coronarien, **caractérisé en ce que** dans la lumière du premier câble d'électrode (12) peut être guidé, en étant déplaçable longitudinalement, et ce au moins sur une section de longueur, un second câble d'électrode (14), qui peut sortir du premier câble d'électrode par une ouverture (52) située sur l'extrémité distale du premier câble d'électrode et est agencé pour être introduit dans une veine cardiaque débouchant dans le sinus coronarien.

2. Dispositif à électrodes selon la revendication 1, **caractérisé en ce que** l'électrode (38,40) du premier câble d'électrode (12) est agencée sous la forme d'une électrode annulaire.

3. Dispositif à électrodes selon la revendication 1 ou 2, **caractérisé en ce que** le premier câble d'électrode (12) comporte deux électrodes distantes axialement (38,40).

4. Dispositif à électrodes selon la revendication 3, **caractérisé en ce que** les deux électrodes (38,40) sont isolées électriquement l'une de l'autre et peuvent être polarisées en des sens opposés.

5. Dispositif à électrodes selon l'une des revendications précédentes, **caractérisé en ce que** le premier câble d'électrode (12) comporte, sur son extrémité distale (52), un dispositif (50) pour la fixation dans le sinus coronarien.

6. Dispositif à électrodes selon l'une des revendications précédentes, **caractérisé en ce que** le premier câble d'électrode (12) se rétrécit sur le côté distal en arrière de l'électrode ou en arrière de l'électrode distale.

7. Dispositif à électrodes selon l'une des revendications précédentes, **caractérisé en ce que** le premier câble d'électrode comporte une gaine extérieure (24) comportant une ouverture de réception (26) pour le second câble d'électrode (14).

8. Dispositif à électrodes selon l'une des revendications précédentes, **caractérisé en ce que** les moyens (30) pour bloquer la position axiale du second câble d'électrode (14) sont prévus pour bloquer la position axiale du second câble d'électrode (14) par rapport à la gaine extérieure (24).

9. Dispositif à électrodes selon la revendication 8, **caractérisé en ce que** les moyens de blocage contiennent un dispositif de serrage (30).

10. Dispositif à électrodes selon la revendication 9, **caractérisé en ce que** le dispositif de serrage est prévu sur l'ouverture de réception (26) et possède une vis excentrique (30), qui s'étend dans la lumière de l'enveloppe extérieure (24) perpendiculairement à l'axe longitudinal entre le premier conducteur d'électrode (12) et le second conducteur d'électrode (14).

11. Dispositif à électrodes selon la revendication 10, **caractérisé en ce que** le profil en coupe transversale de l'enveloppe extérieure (24) s'élargit en direction de l'ouverture de réception (26).

12. Dispositif à électrodes selon l'une des revendications précédentes, **caractérisé en ce que** la lumière du premier câble d'électrode (12) est isolée vis-à-vis de l'entrée et de la sortie d'un liquide et est également isolée électriquement dans la zone de l'électrode ou des électrodes (38,40).

13. Dispositif à électrodes selon la revendication 12, **caractérisé par** une garniture d'étanchéité (52) réalisant une étanchéité vis-à-vis de l'entrée d'un liquide au niveau de l'ouverture distale du premier câble d'électrode (12).

14. Dispositif à électrodes selon l'une des revendications précédentes, **caractérisé en ce que** le second câble d'électrode (14) se rétrécit en direction de son extrémité distale.

15. Dispositif à électrodes selon l'une des revendications précédentes, **caractérisé par** une électrode de pointe (58) située sur l'extrémité distale du second câble d'électrode (14).

16. Dispositif à électrodes selon l'une des revendications précédentes, **caractérisé par** au moins une électrode annulaire dans la section (54) du second câble d'électrode (14), qui ressort distalement de l'enveloppe extérieure du premier câble d'électrode.

17. Dispositif à électrodes selon l'une des revendications précédentes, **caractérisé par** un dispositif (56) pour la fixation du second câble d'électrode (14) dans la veine cardiaque.

18. Dispositif à électrodes selon l'une des revendications précédentes, **caractérisé en ce que** sur l'extrémité distale du second câble d'électrode (14) est disposé un prolongement, notamment commandable, comportant au moins une marque, qui peut être localisée à l'aide d'un procédé d'imagerie à partir de l'extérieur du corps.

19. Dispositif à électrodes selon l'une des revendications précédentes, **caractérisé par** une réalisation bipolaire du second câble d'électrode (14).

20. Dispositif à électrodes selon l'une des revendications précédentes, **caractérisé en ce que** le conducteur d'électrode (32) du premier câble d'électrode (12) et/ou du second câble d'électrode (14) est réalisé sous la forme d'une double hélice.

21. Dispositif à électrodes selon la revendication 20, **caractérisé en ce que** deux fils respectivement isolés électriquement s'étendent dans chacune des deux hélices (46, 48) du conducteur d'électrode (32).

22. Dispositif à électrodes selon l'une des revendications précédentes, **caractérisé en ce que** respectivement un fil de guidage (20,22) est prévu dans la lumière du premier câble d'électrode (32) et dans la lumière d'un conducteur d'électrode du second câble d'électrode (14).

23. Dispositif à électrodes selon l'une des revendications précédentes, **caractérisé en ce que** le second câble d'électrode (14) s'étend entre son extrémité proximale et l'entrée dans l'ouverture de réception du premier câble d'électrode, sous la forme d'une boucle de forme annulaire.
